(19) Europäisches Patentamt
European Patent Office
Office européen des brevets

(11) Veröffentlichungsnummer: **0 334 135**
**A2**

(12) # EUROPÄISCHE PATENTANMELDUNG

(21) Anmeldenummer: 89104338.2

(22) Anmeldetag: 11.03.89

(51) Int. Cl.[4]: C07D 405/04 , A01N 43/64 , C07D 401/04 , C07D 403/04 , C07D 409/04 , C07D 411/04 , C07D 413/04 , C07D 417/04 , A01N 43/647 , A01N 43/74 , A01N 43/80

(30) Priorität: 25.03.88 DE 3810080

(43) Veröffentlichungstag der Anmeldung: 27.09.89 Patentblatt 89/39

(84) Benannte Vertragsstaaten: BE CH DE FR GB IT LI NL

(71) Anmelder: BAYER AG

D-5090 Leverkusen 1 Bayerwerk(DE)

(72) Erfinder: Adler, Alfons, Dr.
Hahnenweg 8
D-5000 Köln 80(DE)
Erfinder: Widdig, Arno, Dr.
Eifgenstrasse 8
D-5068 Odenthal(DE)
Erfinder: Kühle, Engelbert, Dr.
von-Bodelschwingh-Strasse 42
D-5060 Bergisch Gladbach 2(DE)
Erfinder: Führer, Wolfgang, Dr.
Pfarrer-Maybaum-Weg 79
D-5000 Köln 80(DE)
Erfinder: Hagemann, Hermann, Dr.
Kandinskystrasse 52
D-5090 Leverkusen 1(DE)
Erfinder: Hänssler, Gerd, Dr.
Am Arenzberg 58 a
D-5090 Leverkusen 3(DE)

(54) **Trisubstituierte 1,3,5-Triazin-2,4,6-trione.**

(57) Trisubstituierte 1,3,5-Triazin-2,4,6-trione der allgemeinen Formel (I)

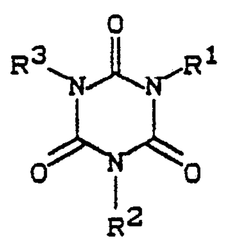

(I),

in welcher
$R^1$, $R^2$ und $R^3$ die in der Beschreibung angegebenen Bedeutungen haben und ihre Verwendung in Schädlingsbekämpfungsmitteln, insbesondere als Fungizide.

Die trisubstituierten 1,3,5-Triazin-2,4,6-trione sind durch die Formel (I) allgemein definiert. Sie können z.B. hergestellt werden, indem man geeignete disubstituierte 1,3,5-Triazin-2,4,6-trione mit geeigneten Halogeniden umsetzt oder indem man geeignete Harnstoffe mit geeigneten Bishalogencarbonylaminen umsetzt.

EP 0 334 135 A2

## Trisubstituierte 1,3,5-Triazin-2,4,6-trione

Die Erfindung betrifft neue trisubstituierte 1,3,5-Triazin-2,4,6-trione, Verfahren zu ihrer Herstellung und ihre Verwendung zur Schädlingsbekämpfung, vor allem als Fungizide.

Es ist bereits bekannt geworden, daß 2-Arylamino-4,6-dichlor-s-triazine, wie z.B. das 4,6-Dichlor-N-(2-chlorphenyl)-1,3,5-triazin-2-amin, fungizide Eigenschaften besitzen (vgl. DAS 1 670 675). Die selektive fungizide Wirksamkeit dieser Stoffe ist jedoch nur auf wenige Pilze beschränkt und nicht immer ausreichend.

Es wurden neue trisubstituierte 1,3,5-Triazin-2,4,6-trione der allgemeinen Formel (I)

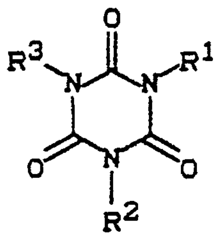

(I),

in welcher

$R^1$ für einen gegebenenfalls substituierten aliphatischen, aromatischen oder cycloaliphatischen Rest steht,

$R^2$ für einen gegebenenfalls substituierten aliphatischen Rest steht und

$R^3$ für gegebenenfalls substituiertes heterocyclisch anelliertes Phenyl steht,

gefunden.

Weiterhin wurde gefunden, daß man die neuen trisubstituierten 1,3,5-Triazin-2,4,6-trione der Formel (I)

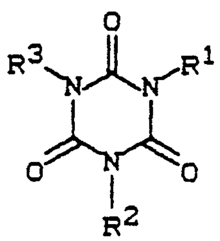

(I)

in welcher

$R^1$ für einen gegebenenfalls substituierten aliphatischen, aromatischen oder cycloaliphatischen Rest steht,

$R^2$ für einen gegebenenfalls substituierten aliphatischen Rest steht und

$R^3$ für gegebenenfalls substituiertes heterocyclisch anelliertes Phenyl steht, erhält, wenn man entweder

a) 1,3-disubstituierte 1,3,5-triazin-2,4,6-trione der allgemeinen Formel (II)

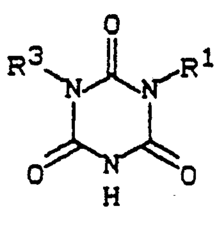

(II),

in welcher

$R^1$ und $R^3$ die oben angegebenen Bedeutungen haben,

mit Verbindungen der allgemeinen Formel (III)

$R^2$-X (III)

in welcher

$R^2$ die oben angegebene Bedeutung hat und

X eine Abgangsgruppe, wie z.B. Halogen oder Sulfat,

bedeutet,
gegebenenfalls in Gegenwart eines Verdünnungsmittels und gegebenenfalls in Gegenwart eines Säurebindemittels umsetzt oder wenn man

b) N,N′-disubstituierte Harnstoffe der allgemeinen Formel (IV)

$R^1$-NH-CO-NH-$R^3$ (IV)

in welcher
$R^1$ und $R^3$ die oben angegebenen Bedeutungen haben, mit einem Bischlorcarbonylamin der allgemeinen Formel

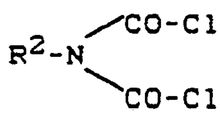

(V)

in welcher
$R^2$ die oben angegebene Bedeutung hat,
gegebenenfalls in Gegenwart eines Verdünnungsmittels und gegebenenfalls in Gegenwart eines Säurebindemittels umsetzt oder wenn man

c) N,N′-disubstituierte Harnstoffe der allgemeinen Formel (VI)

$R^1$-NH-CO-NH-$R^2$ (VI)

in welcher
$R^1$ und $R^2$ die oben angegebene Bedeutung haben,
mit Verbindungen der allgemeinen Formel (VII)

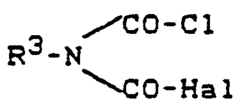

(VII)

in welcher
$R^3$ die oben angegebene Bedeutung hat und
Hal für Halogen, insbesondere Chlor oder Fluor, steht,
umsetzt.

Schließlich wurde gefunden, daß die neuen trisubstituierten 1,3,5-Triazin-2,4,6-trione der Formel (I) sehr gute biologische Eigenschaften besitzen und zur Bekämpfung von Schädlingen, insbesondere von Pilzen und vor allem zur selektiven Bekämpfung von Schadpilzen im Reis geeignet sind.

Die erfindungsgemäßen trisubstituierten 1,3,5-Triazin-2,4,6-trione sind durch die Formel (I) allgemein definiert. Bevorzugt sind Verbindungen der Formel (I), in welcher
$R^1$ für geradkettiges oder verzweigtes Alkyl mit 1 bis 12 Kohlenstoffatomen steht, welches gegebenenfalls ein- bis mehrfach, gleich oder verschieden substituiert ist durch Halogen, Alkoxy mit 1 bis 3 Kohlenstoffatomen, Halogenalkoxy mit 1 oder 2 Kohlenstoffatomen und 1 bis 5 gleichen oder verschiedenen Halogenatomen, Alkylthio mit 1 bis 3 Kohlenstoffatomen, Halogenalkylthio mit 1 oder 2 Kohlenstoffatomen und 1 bis 5 gleichen oder verschiedenen Halogenatomen; für Phenyl steht, welches ebenfalls ein- bis fünffach, gleich oder verschieden substituiert sein kann durch Halogen, Alkyl mit 1 bis 12 Kohlenstoffatomen, Alkoxy mit 1 bis 8 Kohlenstoffatomen; weiterhin für Cycloalkyl mit 3 bis 8 Kohlenstoffatomen steht, welches gegebenenfalls ein- bis sechsfach, gleich oder verschieden substituiert ist durch Halogen, Alkyl mit 1 bis 4 Kohlenstoffatomen, Alkoxy mit 1 bis 3 Kohlenstoffatomen, Halogenalkoxy mit 1 oder 2 Kohlenstoffatomen und 1 bis 5 gleichen oder verschiedenen Halogenatomen, Alkylthio mit 1 bis 3 Kohlenstoffatomen und Halogenalkylthio mit 1 oder 2 Kohlenstoffatomen und 1 bis 5 gleichen oder verschiedenen Halogenatomen,
$R^2$ für Alkyl mit 1 bis 4 Kohlenstoffatomen, Alkenyl mit 3 bis 5 Kohlenstoffatomen, Alkinyl mit 3 bis 5 Kohlenstoffatomen, Alkoxyalkyl mit je 1 bis 3 Kohlenstoffatomen im Alkoxyteil und im Alkylteil, Alkylthioalkyl mit je 1 bis 3 Kohlenstoffatomen im Alkylthioteil und im Alkylteil, Alkoxycarbonylalkyl mit 1 bis 3 Kohlenstoffatomen im Alkoxyteil und 2 oder 3 Kohlenstoffatomen im Alkylteil oder für Cyanoalkyl mit 1 bis 5 Kohlenstoffatomen im Alkylteil steht,
$R^3$ für in 2,3- oder 3,4-Position heterocyclisch anelliertes Phenyl steht, wobei der Heteroring ein oder mehrere, gleiche oder verschiedene Heteroatome enthalten kann und gegebenenfalls ein- bis mehrfach, gleich oder verschieden am isocyclischen und/oder heterocyclischen Ring substituiert ist durch Halogen,

Alkyl mit 1 bis 6 Kohlenstoffatomen, Halogenalkyl mit 1 bis 6 Kohlenstoffatomen und 1 bis 5 gleichen oder verschiedenen Halogenatomen, durch Acyl, welches gegebenenfalls ein- bis mehrfach durch Halogen substituiert ist; durch Alkoxy mit 1 bis 6 Kohlenstoffatomen, Halogenalkoxy mit 1 bis 6 Kohlenstoffatomen und 1 bis 5 gleichen oder verschiedenen Halogenatomen, Alkylthio mit 1 bis 6 Kohlenstoffatomen, Halogenalkylthio mit 1 bis 6 Kohlenstoffatomen und 1 bis 5 gleichen oder verschiedenen Halogenatomen, durch Nitro, durch Dialkylamino mit gleichen oder verschiedenen, geradkettigen oder verzweigten Alkylresten mit je 1 bis 4 Kohlenstoffatomen, Phenyl, welches gegebenenfalls ein- bis fünffach, gleich oder verschieden substi tuiert ist durch Halogen, Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlenstoffatomen, Halogenalkyl mit 1 bis 4 Kohlenstoffatomen und 1 bis 5 gleichen oder verschiedenen Halogenatomen, Alkylthio mit 1 bis 4 Kohlenstoffatomen, Halogenalkylthio mit 1 bis 4 Kohlenstoffatomen und 1 bis 5 gleichen oder verschiedenen Halogenatomen, Nitro und/oder Dialkylamino mit gleichen oder verschiedenen, geradkettigen oder verzweigten Alkylresten mit je 1 bis 4 Kohlenstoffatomen.

Zusätzlich kann der heterocyclische Ring gegebenenfalls durch eine oder mehrere Oxogruppen sein.

Besonders bevorzugt sind Verbindungen der Formel (I), in welcher

$R^1$ für geradkettiges oder verzweigtes Alkyl mit 1 bis 12 Kohlenstoffatomen steht, welches gegebenenfalls ein- bis dreifach, gleich oder verschieden substituiert sein kann durch Fluor, Chlor, Alkoxy mit 1 oder 2 Kohlenstoffatomen, Halogenalkoxy mit 1 oder 2 Kohlenstoffatomen und 1 bis 3 Chlor- und/oder Fluoratomen, Alkylthio mit 1 oder 2 Kohlenstoffatomen, Halogenalkylthio mit 1 oder 2 Kohlenstoffatomen und 1 bis 3 Chlor- und/oder Fluoratomen; für Phenyl steht, welches gegebenenfalls ein- bis dreifach, gleich oder verschieden durch Chlor, Fluor, Alkyl mit 1 bis 8 Kohlenstoffatomen und/oder Alkoxy mit 1 bis 6 Kohlenstoffatomen substituiert sein kann, oder für Cycloalkyl mit 3 bis 6 Kohlenstoffatomen steht, welches gegebenenfalls ein- bis sechsfach, gleich oder verschieden durch Fluor, Chlor, Alkyl mit 1 bis 2 Kohlenstoffatomen, Alkoxy mit 1 oder 2 Kohlenstoffatomen, Halogenalkoxy mit 1 oder 2 Kohlenstoffatomen und 1 bis 5 Chlor- und/oder Fluoratomen, Alkylthio mit 1 oder 2 Kohlenstoffatomen, Halogenalkylthio mit 1 oder 2 Kohlenstoffatomen und mit 1 bis 3 Chlor-und/oder Fluoratomen substituiert sein kann,

$R^2$ für Alkyl mit 1 bis 3 Kohlenstoffatomen, Alkenyl mit 3 oder 4 Kohlenstoffatomen, Alkinyl mit 3 oder 4 Kohlenstoffatomen, Alkoxyalkyl mit 1 oder 2 Kohlenstoffatomen im Alkoxyteil und 1 bis 3 Kohlenstoffatomen im Alkylteil, Alkylthioalkyl mit 1 oder 2 Kohlenstoffatomen im Alkylthioteil und 1 bis 3 Kohlenstoffatomen im Alkylteil, Alkoxycarbonylalkyl mit 1 oder 2 Kohlenstoffatomen im Alkoxyteil und 2 oder 3 Kohlenstoffatomen im Alkylteil oder Cyanoalkyl mit 1 bis 3 Kohlenstoffatomen im Alkylteil steht und

$R^3$ für in 2,3- oder in 3,4-Position heterocyclisch anelliertes Phenyl steht, wobei der Heteroring 5 bis 7 Ringglieder umfassen kann und ein oder mehrere, gleiche oder verschiedene Heteroatome aus der Gruppe Sauerstoff, Schwefel, Stickstoff oder $SO_2$ enthalten kann, und gegebenenfalls im isocyclischen und/oder heterocyclischen Ring ein- bis sechsfach, gleich oder verschieden substituiert sein kann durch Fluor, Chlor, Brom, Alkyl mit 1 bis 4 Kohlenstoffatomen, Halogenalkyl mit 1 bis 3 Kohlenstoffatomen und 1 bis 5 Chlor- und/oder Fluoratomen; durch geradkettiges oder verzweigtes Acyl mit 2 bis 5 Kohlenstoffatomen, welches gegebenenfalls ein- bis fünffach, gleich oder verschieden substituiert ist durch Fluor- und/oder Chloratome; durch Alkoxy mit 1 bis 5 Kohlenstoffatomen, Halogenalkoxy mit 1 oder 2 Kohlenstoffatomen und 1 bis 5 Chlor- und/oder Fluoratomen, Alkylthio mit 1 oder 2 Kohlenstoffatomen, Halogenalkylthio mit 1 oder 2 Kohlenstoffatomen und 1 bis 5 Chlor- und/oder Fluoratomen, Nitro, Dialkylamino mit gleichen oder verschiedenen, geradkettigen oder verzweigten Alkylresten mit je 1 bis 3 Kohlenstoffatomen und/oder Phenyl steht, welches gegebenenfalls ein-bis dreifach, gleich oder verschieden substituiert sein kann durch Fluor, Chlor, Brom, Alkyl mit 1 bis 3 Kohlenstoffatomen, Halogenalkyl mit 1 oder 2 Kohlenstoffatomen und 1 bis 5 Chlor- und/oder Fluoratomen, Alkoxy mit 1 bis 3 Kohlenstoffatomen, Halogenalkoxy mit 1 oder 2 Kohlenstoffatomen und 1 bis 5 Chlor-und/oder Fluoratomen, Alkylthio mit 1 oder 2 Kohlenstoffatomen, Halogenalkylthio mit 1 oder 2 Kohlenstoffatomen und 1 bis 5 Chlor -und/oder Fluoratomen, Nitro und/oder Dialkylamino mit gleichen oder verschiedenen, geradkettigen oder verzweigten Alkylresten mit je 1 bis 3 Kohlenstoffatomen.

Zusätzlich kann der heterocyclische Ring gegebenenfalls durch eine oder mehrere Oxogruppen substituiert sein.

Ganz besonders bevorzugt sind Verbindungen der Formel (I), in denen

$R^1$ für geradkettiges oder verzweigtes Alkyl mit 1 bis 8 Kohlenstoffatomen steht, welches ein- bis dreifach, gleich oder verschieden substituiert sein kann durch Fluor, Chlor, Alkoxy mit 1 oder 2 Kohlenstoffatomen, Alkylthio mit 1 oder 2 Kohlenstoffatomen; für Phenyl steht, welches gegebenenfalls ein- bis dreifach, gleich oder verschieden durch Chlor, Fluor, geradkettiges oder verzweigtes Alkyl mit 1 bis 4 Kohlenstoffatomen

und/oder geradkettiges oder verzweigtes Alkoxy mit 1 bis 5 Kohlenstoffatomen substituiert sein kann, oder für Cycloalkyl mit 3 bis 6 Kohlenstoffatomen steht, welches gegebenenfalls ein- bis dreifach, gleich oder verschieden durch Fluor, Chlor, Methyl, Ethyl, Methoxy, Ethoxy, Methylthio, Ethylthio und/oder Trifluormethyl substituiert sein kann,

$R^2$ für Methyl, Ethyl, Propyl, Allyl, 2-Butenyl, Propargyl, 2-Butinyl, Methoxymethyl, Ethoxymethyl, Methylthiomethyl, Methoxycarbonylmethyl, Ethoxycarbonylmethyl, Methoxycarbonylethyl, Cyanmethyl oder Cyanethyl steht und

$R^3$ für einen der folgenden Heterocyclen steht

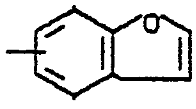 , 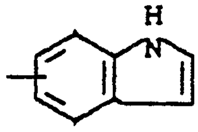 , 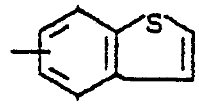 , 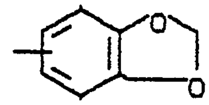

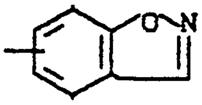 , 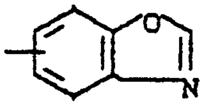 , 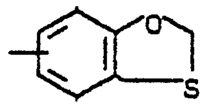 , 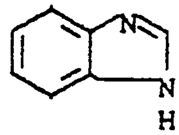

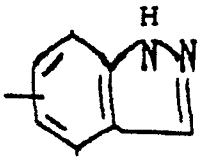 , 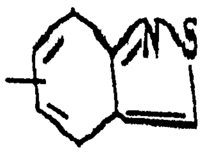 , 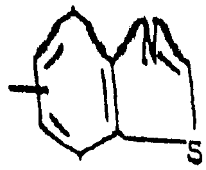 , 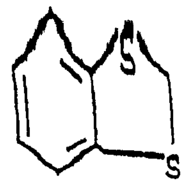

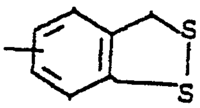 , 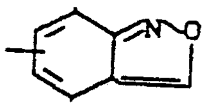 , 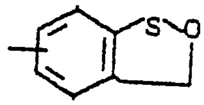 , 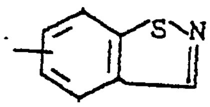

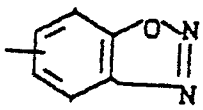 , 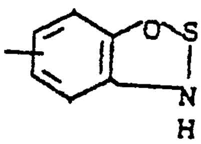 , 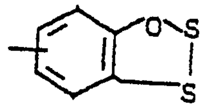 , 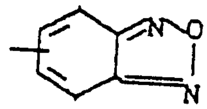

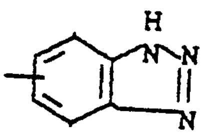 , 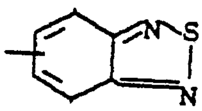 , 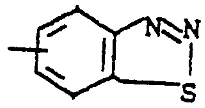 , 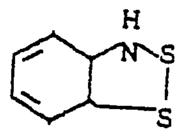

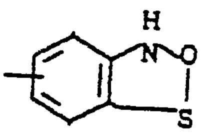 , 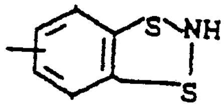 , 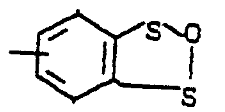 , 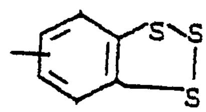

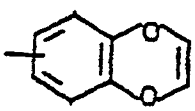 , 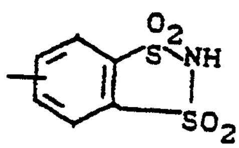 , 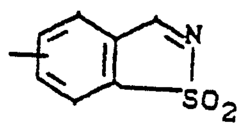 ,

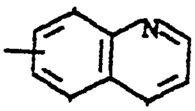 , 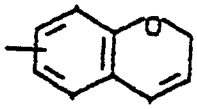 , 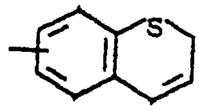

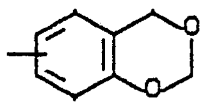 , 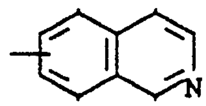 , 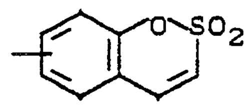 ,

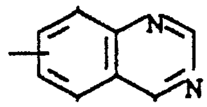 , 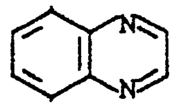 , 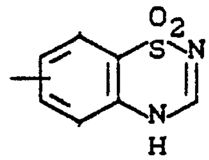

wobei der heterocyclische Ring ganz oder partiell hydriert sein kann und $R^3$ gegebenenfalls im isocyclischen und/oder heterocyclischen Ring ein- bis sechsfach, gleich oder verschieden durch Fluor, Chlor, Niederalkyl mit 1 bis 4 Kohlenstoffatomen, Alkoxy mit 1 bis 4 Kohlenstoffatomen, Trifluormethyl, Acyl mit 2 bis 4 Kohlenstoffatomen, Phenyl, Chlorphenyl und/oder Tolyl substituiert sein kann und der heterocyclische Rest gegebenenfalls durch einen oder mehrere Oxoreste sein kann.

Außerdem sind weiterhin ganz besonders bevorzugt Verbindungen der Formel (I), in denen

$R^1$ für geradkettiges oder verzweigtes Alkyl mit 1 bis 8 Kohlenstoffatomen steht, welches ein- bis dreifach, gleich oder verschieden substituiert sein kann durch Fluor, Chlor, Alkoxy mit 1 oder 2 Kohlenstoffatomen, Alkylthio mit 1 oder 2 Kohlenstoffatomen; für Phenyl steht, welches gegebenenfalls ein- bis dreifach, gleich oder verschieden durch Chlor, Fluor, geradkettiges oder verzweigtes Alkyl mit 1 bis 4 Kohlenstoffatomen und/oder geradkettiges oder verzweigtes Alkoxy mit 1 bis 5 Kohlenstoffatomen substituiert sein kann, oder für Cycloalkyl mit 3 bis 6 Kohlenstoffatomen steht, welches gegebenenfalls ein- bis dreifach, gleich oder verschieden durch Fluor, Chlor, Methyl, Ethyl, Methoxy, Ethoxy, Methylthio, Ethylthio und/oder Trifluormethyl substituiert sein kann,

$R^2$ für Methyl, Ethyl, Propyl, Allyl, 2-Butenyl, Propargyl, 2-Butinyl, Methoxymethyl, 2-Methoxyethyl, Ethoxymethyl, Methylthiomethyl, Methoxycarbonylmethyl, Ethoxycarbonylmethyl, Methoxycarbonylethyl, Cyanmethyl oder Cyanethyl steht und

$R^3$ für die Reste

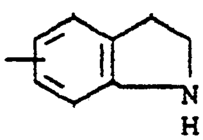 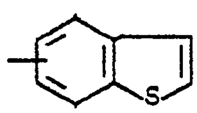 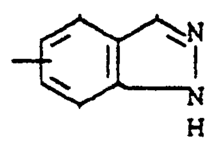 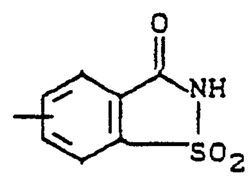 .

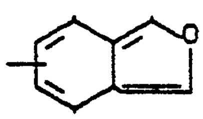 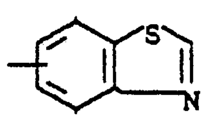 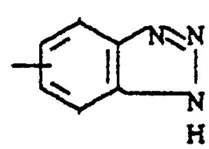 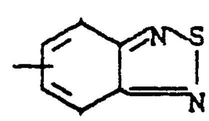

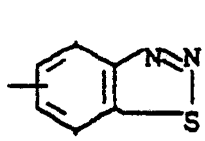 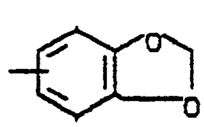 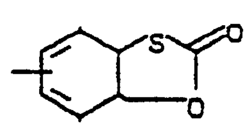 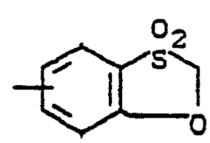

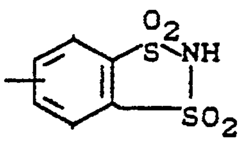
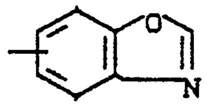
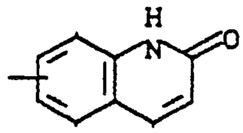
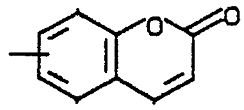

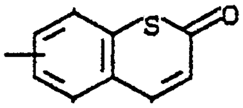
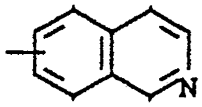
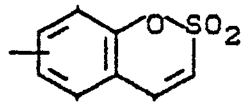

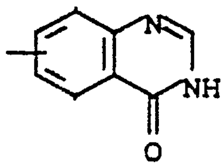
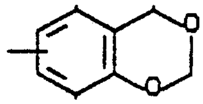
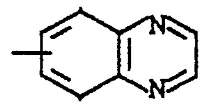

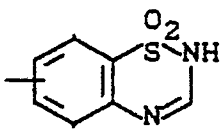
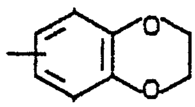

steht, die gegebenenfalls ein- bis fünffach, gleich oder verschieden durch Fluor, Chlor, Methyl, Ethyl, Trifluormethyl, Methoxy, Acetyl, Phenyl oder Tolyl substituiert sind.

Alkyl, Alkoxy, Alkylthio, Halogenalkyl, Halogenalkoxy und Halogenalkylthio können immer geradkettig oder verzweigt sein.

Verwendet man gemäß Verfahren a) 1-(2,2-Dimethylpropyl)-3-(2,2,3,3-tetrafluor-2,3-dihydro-1,4-benzodioxin-6-yl)-1,3,5-triazin-2,4,6-trion und Methyliodid als Ausgangsstoffe, so kann der Reaktionsverlauf durch das folgende Formelschema veranschaulicht werden:

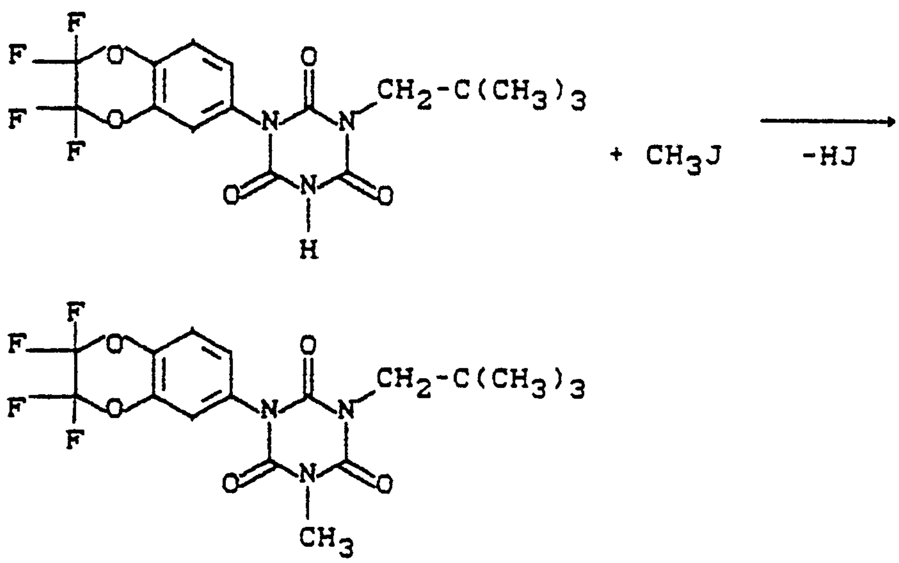


Verwendet man gemäß Verfahren b) N-(2-Phenyl-1,2,3-benzotriazol-5-yl)-N′-(2,2-dimethylpropyl)-harnstoff und Bis-chlorcarbonyl-N-ethylamin als Ausgangsstoffe, so kann der Reaktionsverlauf durch das folgende Formelschema veranschaulicht werden:

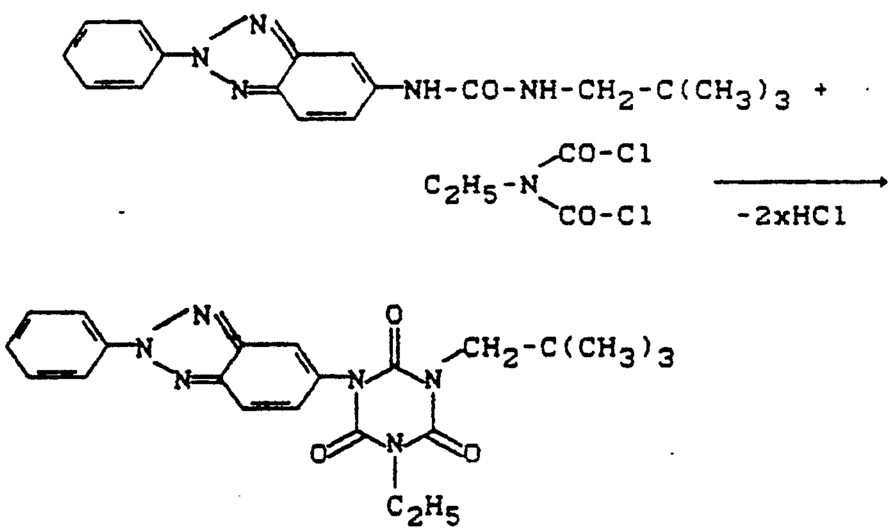


Verwendet man gemäß Verfahren c) Bis-chlorcarbonyl-N-(2,2,3,3-tetrafluor—2,3-dihydro-1,4-benzodioxin-6-yl)-amin und N-Ethyl-N-isopropylharnstoff als Ausgangsstoffe, so kann der Reaktionsverlauf durch das folgende Formelschema veranschaulicht werden:

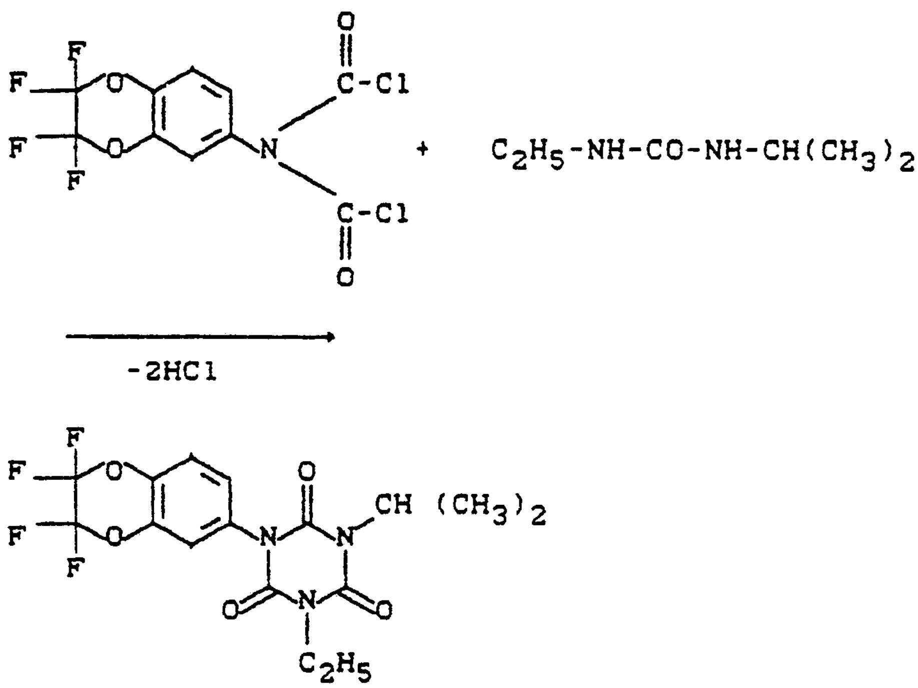


Die bei dem erfindungsgemäßen Verfahren a) als Ausgangsverbindungen benötigten disubstituierten 1,3,5-Triazin-2,4,6-trione sind durch die Formel (II) allgemein definiert. In dieser Formel haben $R^1$ und $R^3$ vorzugsweise diejenige Bedeutung, die bereits im Zusammenhang mit der Beschreibung der erfindungsgemäßen Stoffe der Formel (I) genannt wurde .

Die disubstituierten 1,3,5-Triazin-2,4,6-trione der allgemeinen Formel (II) sind teilweise bekannt oder können in an sich bekannter Weise z.B. aus N,N′-disubstituierten Harnstoffen (IV) und Chlorcarbonylisocyanat erhalten werden (vgl. Angew. Chem. 89 (1977), 789). Die für das Verfahren a) zu verwendenden Alkylierungsmittel der Formel (III) sind ebenfalls bekannt. Bevorzugt steht hierbei X für Chlorid, Bromid, Iodid oder Sulfat.

Die für die erfindungsgemäßen Verfahren b) oder c) zu verwendenden Harnstoffe der allgemeinen Formeln (IV) und (VI) sind an sich bekannt und durch Addition von Isocyanaten an primäre Amine zugänglich (vgl. Houben-Weyl: Methoden der organischen Chemie, Band E 4 (1983) S. 352, Thieme-Verlag,

Stuttgart).

Die nach Verfahren b) oder c) zu verwendenden Bis-chlorcarbonylamine oder Chlor-fluor-carbonylamine der allgemeinen Formeln (V) und (VII) sind ebenfalls bekannt (vgl. Houben-Weyl: Methoden der organischen Chemie, Band E 4 (1983), S. 1022, Thieme-Verlag, Stuttgart).

Die Reaktionstemperaturen können bei den erfindungsgemäßen Verfahren in einem größeren Temperaturbereich variiert werden. Im allgemeinen arbeitet man bei Verfahren a) zwischen 20°C und 150°C, vorzugsweise zwischen 50°C und 120°C, bei den Verfahren b) und c) im allgemeinen zwischen 0°C und 150°C, vorzugsweise zwischen 20°C und 120°C.

Bei der Durchführung der erfindungsgemäßen Verfahren setzt man die Ausgangsstoffe und gegebenenfalls die Säurebindemittel in etwa äquimolaren Mengen ein. Ein Überschuß an säurebindenden Mitteln schadet im allgemeinen nicht.

Die Umsetzungen werden bevorzugt in Gegenwart eines Verdünnungsmittels durchgeführt. Als Verdünnungsmittel kommen alle inerten organischen Lösungsmittel in Frage. Hierzu gehören vorzugsweise Kohlenwasserstoffe wie Toluol und Xylol; chlorierte Kohlenwasserstoffe wie Chlorbenzol und Chloroform; Ketone wie Aceton, Ether wie Tetrahydrofuran und Dioxan; Nitrile wie Acetonitril.

Als Säurebinder können alle üblichen säurebindenden Mittel verwendet werden. Hierzu gehören vorzugsweise tertiäre Amine wie Triethylamin und Pyridin; Alkalihydroxide wie Natrium- und Kaliumhydroxid und Alkalicarbonate und -hydrogencarbonate wie Kaliumcarbonat und Natriumhydrogencarbonat.

Die Aufarbeitung und Isolierung der erfindungsgemäßen Verbindungen erfolgt in üblicher Weise. Sie fallen im allgemeinen sofort kristallin an oder bleiben als Kristallisat nach Verdampfen des Lösungsmittels zurück.

Die erfindungsgemäßen Wirkstoffe weisen eine starke biologische Wirkung auf und können zur Bekämpfung von unerwünschten Schädlingen praktisch eingesetzt werden. Die Wirkstoffe sind für den Gebrauch z. B. als Pflanzenschutzmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft, aber nicht begrenzend seinen einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielseise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen im Mischung mit anderen bekannten Wirkstoffen vorliegen, wie Fungizide, Insektizide, Akarizide und in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Bei entsprechenden Aufwandmengen zeigen die Verbindungen auch eine blattinsektizide Wirkung.

<u>Herstellungsbeispiele</u>

Die Herstellung der erfindungsgemäßen Wirkstoffe nach Verfahren a) geht aus dem nachfolgenden

Beispiel hervor.

Beispiel 1

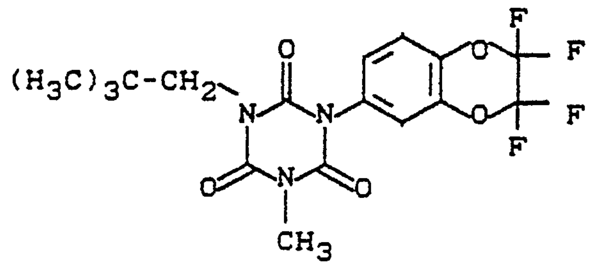

Zu einer Lösung aus 10,0 g (24,7 mMol) 1-(2,2-Dimethylpropyl)-3-(6-(2,2,3,3-tetrafluor-2,3-dihydro-1,4-benzodioxinyl))-1,3,5-triazin-2,4,6-trion und 6,8 g (49 mMol) Kaliumcarbonat in 100 ml Acetonitril werden bei Raumtemperatur 4,2 g (29,6 mMol) Methyliodid getropft. Das Reaktionsgemisch wird 5 Stunden unter Rückfluß gekocht. Nach dem Abkühlen werden die festen Bestandteile abgetrennt und die Mutterlauge in kaltes Wasser gegeben. Die ausgefallenen Kristalle werden abgesaugt und zur Reinigung aus Methanol durch Fällung mit Wasser umkristallisiert. Man erhält auf diese Weise nach Trocknung 9,1 g (88 % der Theorie) an 1-(2,2-Dimethylpropyl)-3-(6-(2,2,3,3-tetrafluor-2,3-dihydro-1,4-benzodioxinyl))-5-methyl-1,3,5-triazin-2,4,6-trion in Form von Kristallen mit einem Schmelzpunkt von 119° C.

Nach der in Beispiel 1 angegebenen Methode oder den allgemeinen Herstellungsangaben werden die in der folgenden Tabelle aufgeführten Stoffe hergestellt:

<u>**T a b e l l e**</u>

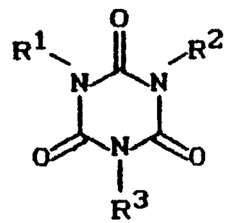 (I)

| Beispiel | $R^3$ | $R^1$ | $R^2$ | Physikal. Konstanten |
|---|---|---|---|---|
| 1 | 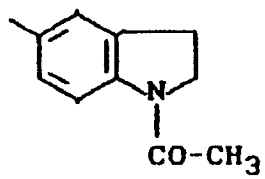 | $-CH(CH_3)_2$ | $-C_2H_5$ | Fp.: 228° C |
| 2 | 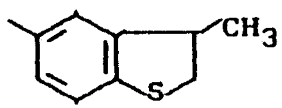 | $-CH(CH_3)_2$ | $-C_2H_5$ | Fp.: 190° C |
| 3 | 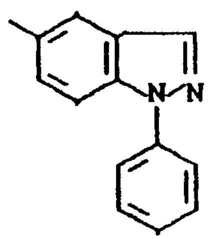 | $-CH(CH_3)_2$ | $-C_2H_5$ | Fp.: 166° C |

<u>T a b e l l e</u> (Fortsetzung)

| Beispiel | $R^3$ | $R^1$ | $R^2$ | Physikal. Konstanten |
|---|---|---|---|---|
| 4 | 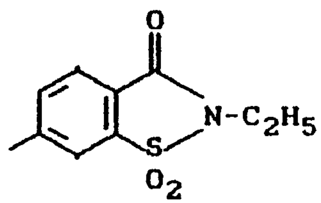 | $-CH(CH_3)_2$ | $-C_2H_5$ | |
| 5 | 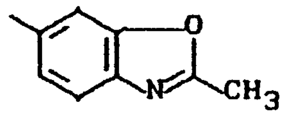 | $-C(CH_3)_3$ | $-C_2H_5$ | |
| 6 | 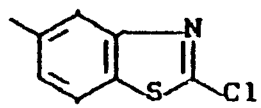 | $-C(CH_3)_3$ | $-C_2H_5$ | Fp.: 106° C |
| 7 | 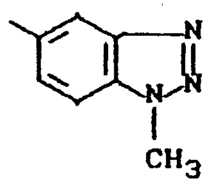 | $-C(CH_3)_3$ | $-C_2H_5$ | Fp.: 196° C |
| 8 | 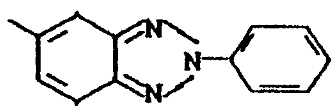 | $-CH_2-C(CH_3)_3$ | $-C_2H_5$ | Fp.: 112° C |

**Tabelle** (Fortsetzung)

| Beispiel | $R^3$ | $R^1$ | $R^2$ | Physikal. Konstanten |
|---|---|---|---|---|
| 9 | 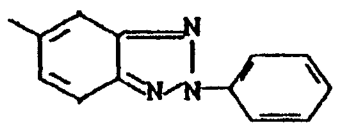 | $-CH_2-C(CH_3)_3$ | $-CH_2-CH_2-CN$ | |
| 10 | " | 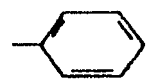 | $-CH_2-CH_2-O-CH_3$ | |
| 11 | " | 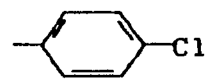 | $-CH_2-CO-O-CH_3$ | |
| 12 | " | 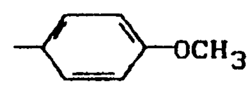 | $-CH_2-CO-O-C_2H_5$ | |
| 13 | " | 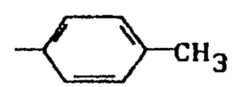 | $-CH_2-CH_2-CO-O-CH_3$ | |

T a b e l l e (Fortsetzung)

| Beispiel | $R^3$ | $R^1$ | $R^2$ | Physikal. Konstanten |
|---|---|---|---|---|
| 14 | 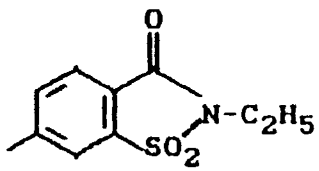 | $-CH(CH_3)_2$ | $-CH_2-CH=CH_2$ | $^1H$-NMR ($CDCl_3$):* 8,15 dd (1H; J=8+0,5 Hz), 7,86 dd (1H; J=2+0,5 Hz), 7,73 dd (1H; J = 8+2 Hz) * ausgewählte Protonen |
| 15 | 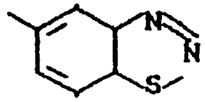 | $-C(CH_3)_3$ | $-CH_3$ | Fp.: 182° C |
| 16 | 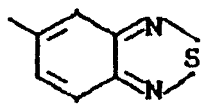 | $-CH(CH_3)_2$ | $-C_2H_5$ | IR: 1682 $cm^{-1}$ |
| 17 | 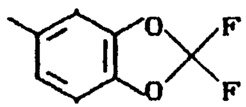 | $-C(CH_3)_3$ | $-CH_3$ | Fp.: 160° C |
| 18 | 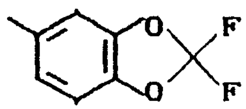 | $-C(CH_3)_3$ | $-C_2H_5$ | Fp.: 136° C |

**T a b e l l e** (Fortsetzung)

| Beispiel | $R^3$ | $R^1$ | $R^2$ | Physikal. Konstanten |
|---|---|---|---|---|
| 19 | 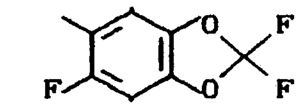 | $-C(CH_3)_3$ | $-C_2H_5$ | Fp.: 98° C |
| 20 | 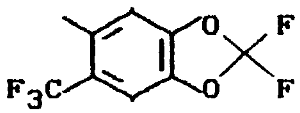 | $-C(CH_3)_3$ | $-C_2H_5$ | Fp.: 152° C |
| 21 | 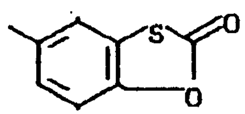 | $-CH(CH_3)_2$ | $-C_2H_5$ | IR: 1683 $cm^{-1}$ |
| 22 | 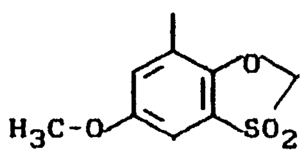 | $-CH(CH_3)_2$ | $-C_2H_5$ | Fp.: 210° C |
| 23 | 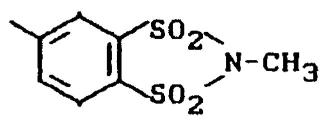 | $-CH(CH_3)_2$ | $-CH_2-C{\equiv}CH$ | Fp.: 134° C |

**Tabelle** (Fortsetzung)

| Beispiel | $R^3$ | $R^1$ | $R^2$ | Physikal. Konstanten |
|---|---|---|---|---|
| 24 | 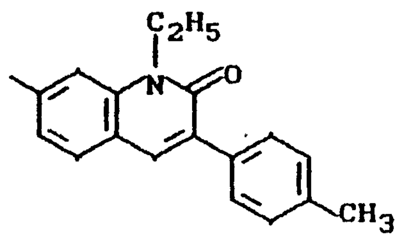 | $-C(CH_3)_3$ | $-CH_3$ | Fp.: 252° C |
| 25 | 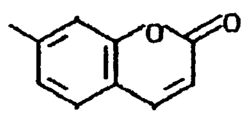 | $-CH(CH_3)_2$ | $-CH_2-CH=CH_2$ | Fp.: 204° C |
| 26 | 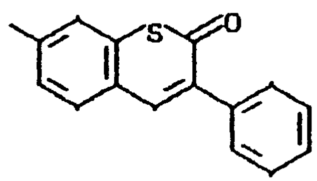 | $-CH(CH_3)_2$ | $-C_2H_5$ | Fp.: > 260° C |
| 27 | 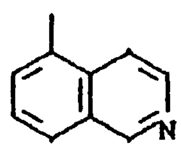 | $-CH(CH_3)_2$ | $-CH_3$ | Fp.: 142° C |

**T a b e l l e** (Fortsetzung)

| Beispiel | $R^3$ | $R^1$ | $R^2$ | Physikal. Konstanten |
|---|---|---|---|---|
| 28 | 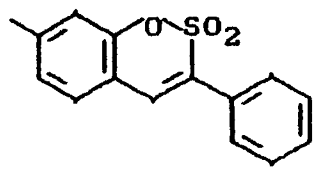 | $-CH(CH_3)_2$ | $-CH_3$ | IR: 1682 $cm^{-1}$ |
| 29 | 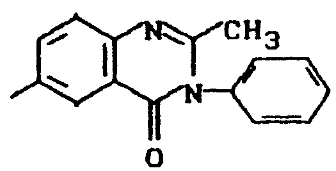 | $-C(CH_3)_3$ | $-CH_3$ | $^1$H-NMR (DMSO): 8,10 dd (1H; J = 2+1Hz), 7,74 m (2H), 7,60-7,50 m (3H), 7,46 d (2H; J= 8 Hz), 3,17 s (3H), 2.15 s (3H), 1,62 s (9H). |
| 30 | 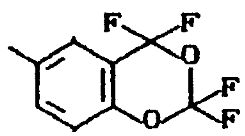 | $-C(CH_3)_3$ | $-C_2H_5$ | Fp.: 114° C |
| 31 | 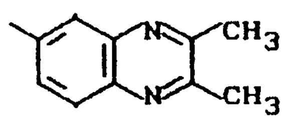 | $-CH(CH_3)_2$ | $-CH_3$ | Fp.: 156° C |

**Tabelle** (Fortsetzung)

| Beispiel | $R^3$ | $R^1$ | $R^2$ | Physikal. Konstanten |
|---|---|---|---|---|
| 32 | 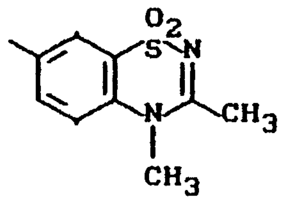 | $-CH(CH_3)_2$ | $-C_2H_5$ | Fp.: > 260° C |
| 33 | 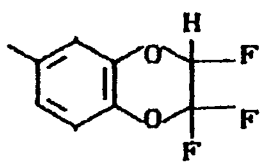 | $-C(CH_3)_3$ | $-C_2H_5$ | Fp.: 132° C |
| 34 | " | " | $-C_3H_7$-n | Fp.: 168° C |
| 35 | 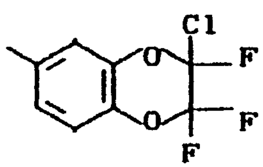 | $-C(CH_3)_3$ | $-CH_3$ | Fp.: 102° C |
| 36 | " | " | $-C_2H_5$ | Fp.: 52° C |

T a b e l l e (Fortsetzung)

| Beispiel | $R^3$ | $R^1$ | $R^2$ | Physikal. Konstanten |
|---|---|---|---|---|
| 37 | 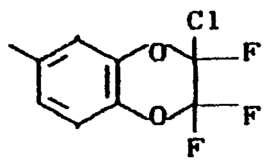 | $-C(CH_3)_3$ | $-CH_2-C{\equiv}CH$ | IR: 1698 $cm^{-1}$ |
| 38 | " | " | $-CH_2-CH=CH_2$ | IR: 1700 $cm^{-1}$ |
| 39 | 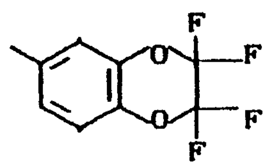 | $-CH(CH_3)_2$ | $-C_2H_5$ | Fp.: 102° C |
| 40 | " | " | $-CH_2-CH=CH_2$ | Fp.: 92° C |
| 41 | " | $-C(CH_3)_3$ | $-CH_3$ | Fp.: 128° C |
| 42 | " | " | $-C_2H_5$ | Fp.: 128° C |
| 43 | " | " | $-CH_2-C{\equiv}CH$ | IR: 1698 $cm^{-1}$ |

T a b e l l e (Fortsetzung)

| Beispiel | $R^3$ | $R^1$ | $R^2$ | Physikal. Konstanten |
|---|---|---|---|---|
| 44 | 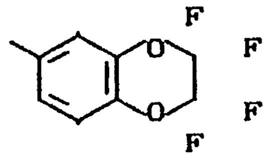 | $-C(CH_3)_3$ | $-CH_2-CH=CH_2$ | IR: 1695 $cm^{-1}$ |
| 45 | " | " | $-CH_2-CH,Z,E=CH-CH_3$ | Z/E 1/5<br>$^1H$-NMR($CDCl_3$):4,53 dt (2H;J=6+1Hz; Z);<br>4,40 dt (2H;J=6+$_1$HZ,E) |
| 46 | " | $-C(CH_3)_3$ | $-CH_2-CH=CH_2$ | IR $CHCl_3$: 1690 $cm^{-1}$ |
| 47 | " | $-CH_2-C(CH_3)_3$ | $-CH_3$ | IR: 1698 $cm^{-1}$ |
| 48 | " | " | $-CH_2-CH=CH_2$ | IR $CHCl_3$: 1690 $cm^{-1}$ |
| 49 | " | 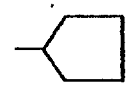 | $-C_2H_5$ | IR: 1697 $cm^{-1}$ |
| 50 | " | " | $-CH_2-CH=CH_2$ | IR: 1695 $cm^{-1}$ |
| 51 | " | 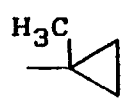 | $-C_2H_5$ | Fp.: 114° C |

T a b e l l e (Fortsetzung)

| Beispiel | $R^3$ | $R^1$ | $R^2$ | Physikal. Konstanten |
|---|---|---|---|---|
| 52 | 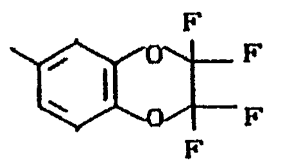 | $H_3C$ | $-CH_2-CH=CH_2$ | Fp.: 116° C |
| 53 | 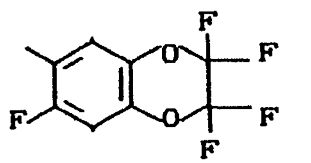 | $-C(CH_3)_3$ | $-CH_3$ | Fp.: 122° C |
| 54 | 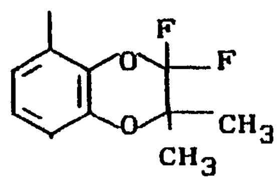 | $-C(CH_3)_3$ | $-C_2H_5$ | Fp.: 144° C |
| 55 | 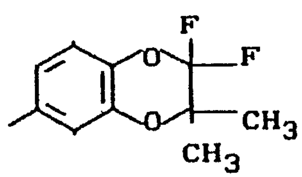 | $-C(CH_3)_3$ | $-C_2H_5$ | Fp.: 108° C |

T a b e l l e (Fortsetzung)

| Beispiel | $R^3$ | $R^1$ | $R^2$ | Physikal. Konstanten |
|---|---|---|---|---|
| 56 | 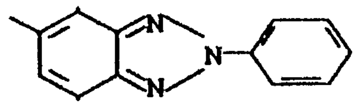 | $-CH_2-C(CH_3)_3$ | $-C_2H_5$ | Fp.: 112° C |
| 57 | " | 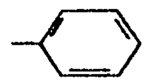 | $-CH_2COOC_2H_5$ | Fp.: 212° C |
| 58 | " | 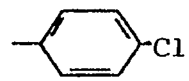 | $-CH_2CH_2-CN$ | Fp.: 165° C |
| 59 | " | 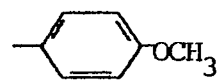 | $-CH_2CH_2-O-CH_3$ | Fp.: 94-96° C |
| 60 | " | 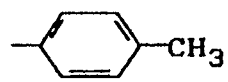 | $-CH_2CH_2-COOC_2H_5$ | Fp.: 92° C |
| 61 | 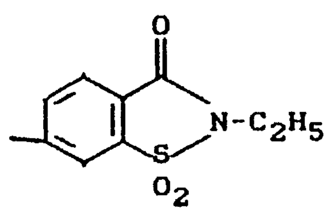 | $-CH(CH_3)_2$ | $-CH_2-CH=CH_2$ | Fp.: 87° C |

Anwendungsbeispiele

Beispiel A

Pyricularia-Test (Reis) /protektiv

| | | |
|---|---|---|
| Lösungsmittel: | 12,5 | Gewichtsteile Aceton |
| Emulgator: | 0,3 | Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Viele der Beispiele zeigen bei einer Wirkstoffkonzentration von 0,025 % einen Wirkungsgrad zwischen 90 und 100 % gegenüber der unbehandelten Kontrolle.

Beispiel B

Pyricularia-Test (Reis) / systemisch

| | | |
|---|---|---|
| Lösungsmittel: | 12,5 | Gewichtsteile Aceton |
| Emulgator: | 0,3 | Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Krankheitsbefalls.

Die meisten der Verbindungen zeigen z.B. bei einer Aufwandmenge von 100 mg Wirkstoff pro 100 $cm^2$ einen Wirkungsgrad zwischen 80 und 86 %.

## Ansprüche

1. Trisubstituierte 1,3,5-Triazin-2,4,6-trione der allgemeinen Formel (I)

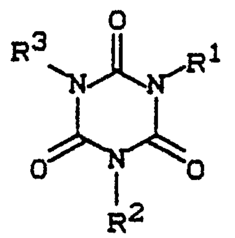

(I)

in welcher
$R^1$ für einen gegebenenfalls substituierten aliphatischen, aromatischen oder cycloaliphatischen Rest steht,
$R^2$ für einen gegebenenfalls substituierten aliphatischen Rest steht und
$R^3$ für gegebenenfalls substituiertes heterocyclisch anelliertes Phenyl steht.

2. Trisubstituierte 1,3,5-Triazin-2,4,6-trione gemäß Anspruch 1, wobei in der Formel (I)
$R^1$ für geradkettiges oder verzweigtes Alkyl mit 1 bis 12 Kohlenstoffatomen steht, welches gegebenenfalls ein- bis mehrfach, gleich oder verschieden substituiert ist durch Halogen, Alkoxy mit 1 bis 3 Kohlenstoffatomen, Halogenalkoxy mit 1 oder 2 Kohlenstoffatomen und 1 bis 5 gleichen oder verschiedenen Halogenatomen, Alkylthio mit 1 bis 3 Kohlenstoffatomen, Halogenalkylthio mit 1 oder 2 Kohlenstoffatomen und 1 bis 5 gleichen oder verschiedenen Halogenatomen; für Phenyl steht, welches ebenfalls ein- bis fünffach, gleich oder verschieden substituiert sein kann durch Halogen, Alkyl mit 1 bis 12 Kohlenstoffatomen, Alkoxy mit 1 bis 8 Kohlenstoffatomen; weiterhin für Cycloalkyl mit 3 bis 8 Kohlenstoffatomen steht, welches gegebenenfalls ein- bis sechsfach, gleich oder verschieden substituiert ist durch Halogen, Alkyl mit 1 bis 4 Kohlenstoffatomen, Alkoxy mit 1 bis 3 Kohlenstoffatomen, Halogenalkoxy mit 1 oder 2 Kohlenstoffatomen und 1 bis 5 gleichen oder verschiedenen Halogenatomen, Alkylthio mit 1 bis 3 Kohlenstoffatomen und Halogenalkylthio mit 1 oder 2 Kohlenstoffatomen und 1 bis 5 gleichen oder verschiedenen Halogenatomen,
$R^2$ für Alkyl mit 1 bis 4 Kohlenstoffatomen, Alkenyl mit 3 bis 5 Kohlenstoffatomen, Alkinyl mit 3 bis 5 Kohlenstoffatomen, Alkoxyalkyl mit je 1 bis 3 Kohlenstoffatomen im Alkoxyteil und im Alkylteil, Alkylthioalkyl mit je 1 bis 3 Kohlenstoffatomen im Alkylthioteil und im Alkylteil, Alkoxycarbonylalkyl mit 1 bis 3 Kohlenstoffatomen im Alkoxyteil und 2 oder 3 Kohlenstoffatomen im Alkylteil oder für Cyanoalkyl mit 1 bis 5 Kohlenstoffatomen im Alkylteil steht,
$R^3$ für in 2,3- oder 3,4-Position heterocyclisch anelliertes Phenyl steht, wobei der Heteroring ein oder mehrere, gleiche oder verschiedene Heteroatome enthalten kann und gegebenenfalls ein- bis mehrfach, gleich oder verschieden am isocyclischen und/oder heterocyclischen Ring substituiert ist durch Halogen, Alkyl mit 1 bis 6 Kohlenstoffatomen, Halogenalkyl mit 1 bis 6 Kohlenstoffatomen und 1 bis 5 gleichen oder verschiedenen Halogenatomen, durch Acyl, welches gegebenenfalls ein- bis mehrfach durch Halogen substituiert ist; durch Alkoxy mit 1 bis 6 Kohlenstoffatomen, Halogenalkoxy mit 1 bis 6 Kohlenstoffatomen und 1 bis 5 gleichen oder verschiedenen Halogenatomen, Alkylthio mit 1 bis 6 Kohlenstoffatomen, Halogenalkylthio mit 1 bis 6 Kohlenstoffatomen und 1 bis 5 gleichen oder verschiedenen Halogenatomen, durch Nitro, durch Dialkylamino mit gleichen oder verschiedenen, geradkettigen oder verzweigten Alkylresten mit je 1 bis 4 Kohlenstoffatomen, Phenyl, welches gegebenenfalls ein- bis fünffach, gleich oder verschieden substituiert ist durch Halogen, Alkyl mit 1 bis 4 Kohlenstoffatomen, Halogenalkyl mit 1 bis 4 Kohlenstoffatomen und 1 bis 5 gleichen oder verschiedenen Halogenatomen, Alkoxy mit 1 bis 4 Kohlen stoffatomen Halogenalkyl mit 1 bis 4 Kohlenstoffatomen und 1 bis 5 gleichen oder verschiedenen Halogenatomen, Alkylthio mit 1 bis 4 Kohlenstoffatomen, Halogenalkylthio mit 1 bis 4 Kohlenstoffatomen und 1 bis 5 gleichen oder verschiedenen Halogenatomen, Nitro und/oder Dialkylamino mit gleichen oder verschiedenen, geradkettigen oder verzweigten Alkylresten mit je 1 bis 4 Kohlenstoffatomen.
Zusätzlich kann der heterocyclische Ring gegebenenfalls durch eine oder mehrere Oxogruppen substiutiert sein.

3. Trisubstituierte 1,3,5-Triazin-2,4,6-trione gemäß Anspruch 1, wobei in der Formel (I)
$R^1$ für geradkettiges oder verzweigtes Alkyl mit 1 bis 12 Kohlenstoffatomen steht, welches gegebenenfalls ein- bis dreifach, gleich oder verschieden substituiert sein kann durch Fluor, Chlor, Alkoxy mit 1 oder 2 Kohlenstoffatomen, Halogenalkoxy mit 1 oder 2 Kohlenstoffatomen und 1 bis 3 Chlor-und/oder Fluoratomen, Alkylthio mit 1 oder 2 Kohlenstoffatomen, Halogenalkylthio mit 1 oder 2 Kohlenstoffatomen und 1 bis 3 Chlor-und/oder Fluoratomen; für Phenyl steht, welches gegebenenfalls ein- bis dreifach, gleich oder

verschieden durch Chlor, Fluor, Alkyl mit 1 bis 8 Kohlenstoffatomen und/oder Alkoxy mit 1 bis 6 Kohlenstoffatomen substituiert sein kann, oder für Cycloalkyl mit 3 bis 6 Kohlenstoffatomen steht, welches gegebenenfalls ein-bis sechsfach, gleich oder verschieden durch Fluor, Chlor, Alkyl mit 1 oder 2 Kohlenstoffatomen, Alkoxy mit 1 oder 2 Kohlenstoffatomen, Halogenalkoxy mit 1 oder 2 Kohlenstoffatomen und 1 bis 5 Chlor- und/oder Fluoratomen, Alkylthio mit 1 oder 2 Kohlenstoffatomen, Halogenalkylthio mit 1 oder 2 Kohlenstoffatomen und mit 1 bis 3 Chlor-und/oder Fluoratomen substituiert sein kann,

$R^2$ für Alkyl mit 1 bis 3 Kohlenstoffatomen, Alkenyl mit 3 oder 4 Kohlenstoffatomen, Alkinyl mit 3 oder 4 Kohlenstoffatomen, Alkoxyalkyl mit 1 oder 2 Kohlenstoffatomen im Alkoxyteil und 1 bis 3 Kohlenstoffatomen im Alkylteil, Alkylthioalkyl mit 1 oder 2 Kohlenstoffatomen im Alkylthioteil und 1 bis 3 Kohlenstoffatomen im Alkylteil, Alkoxycarbonylalkyl mit 1 oder 2 Kohlenstoffatomen im Alkoxyteil und 2 oder 3 Kohlenstoffatomen im Alkylteil oder Cyanoalkyl mit 1 bis 3 Kohlenstoffatomen im Alkylteil steht und

$R^3$ für in 2,3- oder in 3,4-Position heterocyclisch anelliertes Phenyl steht, wobei der Heteroring 5 bis 7 Ringglieder umfassen kann und ein oder mehrere, gleiche oder verschiedene Heteroatome aus der Gruppe Sauerstoff, Schwefel, Stickstoff oder $SO_2$ enthalten kann, und gegebenenfalls im isocyclischen und/oder heterocyclischen Ring ein- bis sechsfach, gleich oder verschieden substituiert sein kann durch Fluor, Chlor, Brom, Alkyl mit 1 bis 4 Kohlenstoffatomen, Halogenalkyl mit 1 bis 3 Kohlenstoffatomen und 1 bis 5 Chlor- und/oder Fluoratomen; durch geradkettiges oder verzweigtes Acyl mit 2 bis 5 Kohlenstoffatomen, welches gegebenenfalls ein- bis fünffach, gleich oder verschieden substituiert ist durch Fluor- und/oder Chloratome; durch Alkoxy mit 1 bis 5 Kohlenstoffatomen, Halogen alkoxy mit 1 oder 2 Kohlenstoffatomen und 1 bis 5 Chlor- und/oder Fluoratomen, Alkylthio mit 1 oder 2 Kohlenstoffatomen, Halogenalkylthio mit 1 oder 2 Kohlenstoffatomen und 1 bis 5 Chlor-und/oder Fluoratomen, Nitro, Dialkylamino mit gleichen oder verschiedenen, geradkettigen oder verzweigten Alkylresten mit je 1 bis 3 Kohlenstoffatomen und/oder Phenyl steht, welches gegebenenfalls ein- bis dreifach, gleich oder verschieden substituiert sein kann durch Fluor, Chlor, Brom, Alkyl mit 1 bis 3 Kohlenstoffatomen, Halogenalkyl mit 1 oder 2 Kohlenstoffatomen und 1 bis 5 Chlor- und/oder Fluoratomen, Alkoxy mit 1 bis 3 Kohlenstoffatomen, Halogenalkoxy mit 1 oder 2 Kohlenstoffatomen und 1 bis 5 Chlor-und/oder Fluoratomen, Alkylthio mit 1 oder 2 Kohlenstoffatomen, Halogenalkylthio mit 1 oder 2 Kohlenstoffatomen und 1 bis 5 Chlor -und/oder Fluoratomen, Nitro und/oder Dialkylamino mit gleichen oder verschiedenen, geradkettigen oder verzweigten Alkylresten mit je 1 bis 3 Kohlenstoffatomen. Zusätzlich kann der heterocyclische Ring gegebenenfalls durch eine oder mehrere Oxogruppen substituiert sein.

4. Trisubstituierte 1,3,5-Triazin-2,4,6-trione gemäß Anspruch 1, wobei in der Formel (I)

$R^1$ für geradkettiges oder verzweigtes Alkyl mit 1 bis 8 Kohlenstoffatomen steht, welches ein-bis dreifach, gleich oder verschieden substituiert sein kann durch Fluor, Chlor, Alkoxy mit 1 oder 2 Kohlenstoffatomen, Alkylthio mit 1 oder 2 Kohlenstoffatomen, für Phenyl steht, welches gegebenenfalls ein- bis dreifach, gleich oder verschieden durch Chlor, Fluor, geradkettiges oder verzweigtes Alkyl mit 1 bis 4 Kohlenstoffatomen und/oder geradkettiges oder verzweigtes Alkoxy mit 1 bis 5 Kohlenstoffatomen substituiert sein kann, oder für Cycloalkyl mit 3 bis 6 Kohlenstoffatomen steht, welches gegebenenfalls ein- bis dreifach, gleich oder verschieden durch Fluor, Chlor, Methyl, Ethyl, Methoxy, Ethoxy, Methylthio, Ethylthio und/oder Trifluormethyl substituiert sein kann,

$R^2$ für Methyl, Ethyl, Propyl, Allyl, 2-Butenyl, Propargyl, 2-Butinyl, Methoxymethyl, Ethoxymethyl, Methylthiomethyl, Methoxycarbonylmethyl, Ethoxycarbonylmethyl, Methoxycarbonylethyl, Cyanmethyl oder Cyanethyl steht und

$R^3$ für einen der folgenden Heterocyclen steht,

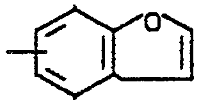 , 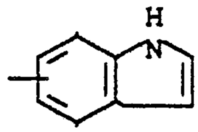 , 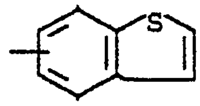 , 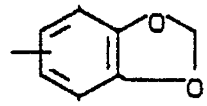

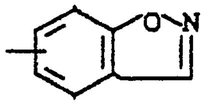 , 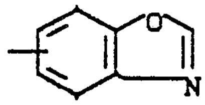 , 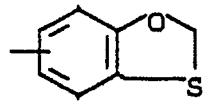 , 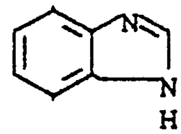

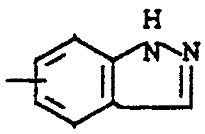 , 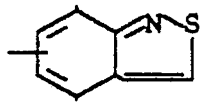 , 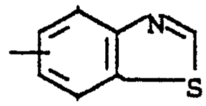 , 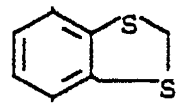

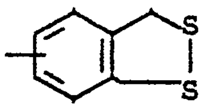 , 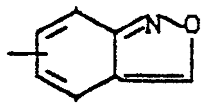 , 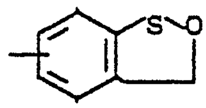 , 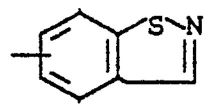

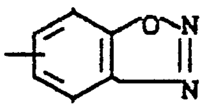 , 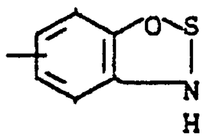 , 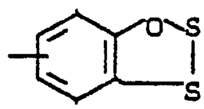 , 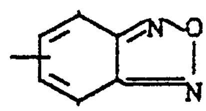

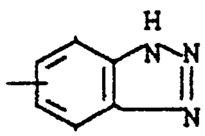 , 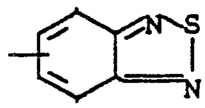 , 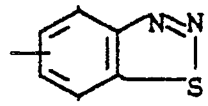 , 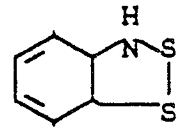

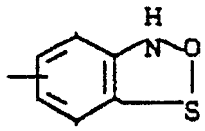 , 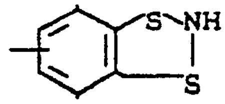 , 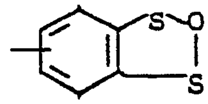 , 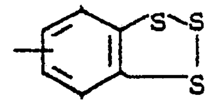

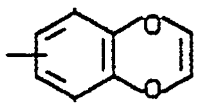 , 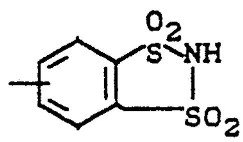 , 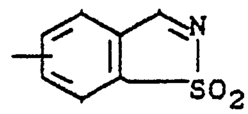 ,

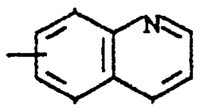 , 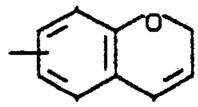 , 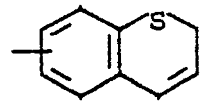

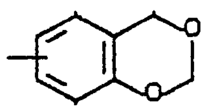 , 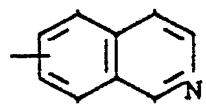 , 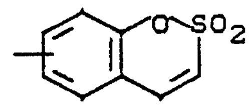 ,

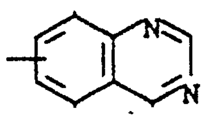 , 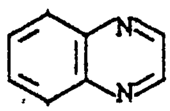 , 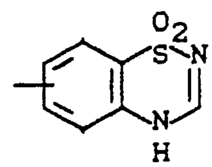

wobei der heterocyclische Ring ganz oder partiell hydriert sein kann und $R^3$ gegebenenfalls im isocyclischen und/oder heterocyclischen Ring ein- bis sechsfach, gleich oder verschieden durch Fluor, Chlor, Niederalkyl mit 1 bis 4 Kohlenstoffatomen, Alkoxy mit 1 bis 4 Kohlenstoffatomen, Trifluormethyl, Acyl mit 2 bis 4 Kohlenstoffatomen, Phenyl, Chlorphenyl und/oder Tolyl substituiert sein kann und der heterocyclische Rest gegebenenfalls durch einen oder mehrere Oxoreste zusätzlich substituiert sein kann.

5. Trisubstituierte 1,3,5-Triazin-2,4,6-trione gemäß Anspruch 1, wobei in der Formel (I)
$R^1$ für geradkettiges oder verzweigtes Alkyl mit 1 bis 8 Kohlenstoffatomen steht, welches ein-bis dreifach, gleich oder verschieden substituiert sein kann durch Fluor, Chlor, Alkoxy mit 1 oder 2 Kohlenstoffatomen, Alkylthio mit 1 oder 2 Kohlenstoffatomen; für Phenyl steht, welches gegebenenfalls ein- bis dreifach, gleich oder verschieden durch Chlor, Fluor, geradkettiges oder verzweigtes Alkyl mit 1 bis 4 Kohlenstoffatomen und/oder geradkettiges oder verzweigtes Alkoxy mit 1 bis 5 Kohlenstoffatomen substituiert sein kann, oder für Cycloalkyl mit 3 bis 6 Kohlenstoffatomen steht, welches gegebenenfalls ein- bis dreifach, gleich oder verschieden durch Fluor, Chlor, Methyl, Ethyl, Methoxy, Ethoxy, Methylthio, Ethylthio und/oder Trifluormethyl substituiert sein kann,
$R^2$ für Methyl, Ethyl, Propyl, Allyl, 2-Butenyl, Propargyl, 2-Butinyl, Methoxymethyl, 2-Methoxyethyl, Ethoxymethyl, Methylthiomethyl, Methoxycarbonylmethyl, Ethoxycarbonylmethyl, Methoxycarbonylethyl, Cyanmethyl oder Cyanethyl steht und
$R^3$ für die Reste

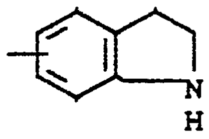 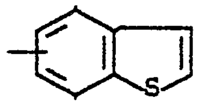 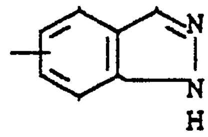 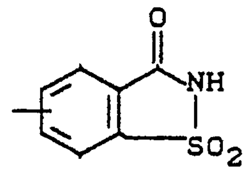

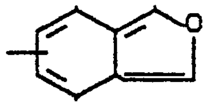 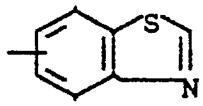 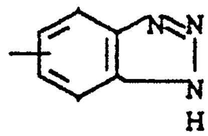 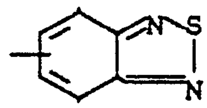

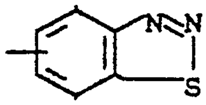 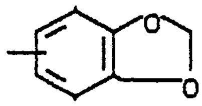 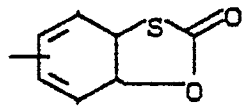 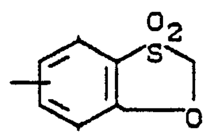

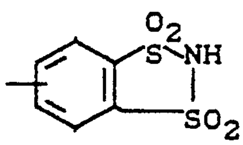 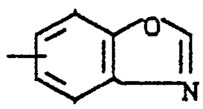 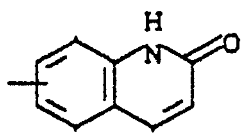 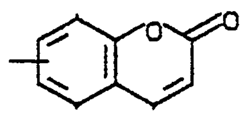

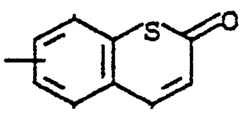 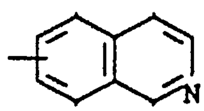 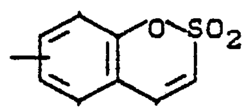

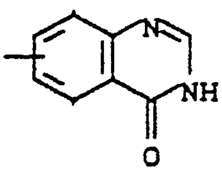 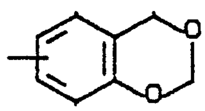 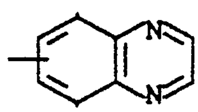

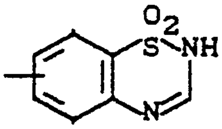 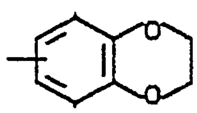

steht, die gegebenenfalls ein- bis fünffach, gleich oder verschieden durch Fluor, Chlor, Methyl, Ethyl, Trifluormethyl, Methoxy, Acetyl, Phenyl oder Tolyl substituiert sind.

6. Verfahren zur Herstellung von trisubstituierten 1,3,5-Triazin-2,4,6-trionen der allgemeinen Formel (I)

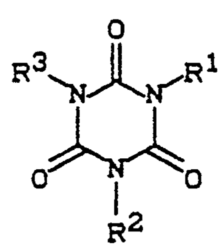 (I)

in welcher

$R^1$ für einen gegebenenfalls substituierten aliphatischen, aromatischen oder cycloaliphatischen Rest steht und

$R^2$ für einen gegebenenfalls substituierten aliphatischen Rest steht und
$R^3$ für gegebenenfalls substituiertes heterocyclisch anelliertes Phenyl steht,
dadurch gekennzeichnet, daß man
a) 1,3-disubstituierte 1,3,5-Triazin-2,4,6-trione der allgemeinen Formel (II)

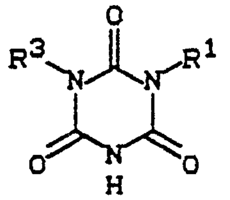

(II),

in welcher
$R^1$ and $R^3$ die oben angegebenen Bedeutungen haben,
mit Verbindungen der allgemeinen Formel (III)
$R^2$ - X (III),
in welcher
$R^2$ die oben angegebene Bedeutung hat und
X eine Abgangsgruppe
bedeutet,
gegebenenfalls in Gegenwart eines Verdünnungsmittels und gegebenenfalls in Gegenwart eines Säurebindemittels umsetzt oder
b) N,N′-disubstituierte Harnstoffe der allgemeinen Formel (IV)
$R^1$-NH-CO-NH-$R^3$ (IV)
in welcher
$R^1$ und $R^3$ die oben angegebenen Bedeutungen haben, mit einem Bischlorcarbonylamin der allgemeinen Formel

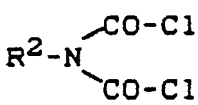

(V),

gegebenenfalls in Gegenwart eines Verdünnungsmittels und gegebenenfalls in Gegenwart eines Säurebindemittels umsetzt oder
c) N,N′-disubstituierte Harnstoffe der allgemeinen Formel (VI)
$R^1$-NH-CO-NH-$R^{2'}$ (VI),
in welcher
$R^1$ und $R^2$ die oben angegebene Bedeutung haben mit Verbindungen der allgemeinen Formel (VII)

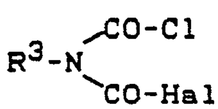

(VII)

in welcher
$R^3$ die oben angegebene Bedeutung hat und
Hal für Halogen steht,
umsetzt.

7. Schädlingsbekämpfungsmittel, gekennzeichnet durch einen Gehalt an mindestens einem trisubstituierten 1,3,5-Triazin-2,4,6-trion der Formel (I) gemäß den Ansprüchen 1 bis 6.

8. Verwendung von trisubstituierten 1,3,5-Triazin-2,4,6-trionen der Formel (I) nach den Ansprüchen 1 bis 6 zur Bekämpfung von Schädlingen.

9. Verfahren zur Bekämpfung von Schädlingen, dadurch gekennzeichnet, daß man trisubstituierte 1,3,5-Triazin-2,4,6-trione der Formel (I) nach den Ansprüchen 1 bis 6 auf Schädlinge und/oder ihren Lebensraum einwirken läßt.

10. Verfahren zur Herstellung von Schädlingsbekämpfungsmitteln, dadurch gekennzeichnet, daß man trisubstituierte 1,3,5-Triazin-2,4,6-trione der Formel (I) nach den Ansprüchen 1 bis 6 mit Streckmitteln und/oder oberflächenaktiven Mitteln vermischt.